# EUROPEAN PATENT SPECIFICATION

(11) **EP 0 994 099 B1**
(45) Date of publication and mention of the grant of the patent: **02.02.2005**
(21) Application number: 98420187.1
(22) Date of filing: 13.10.1998
(51) Int. Cl.: C07C 201/12, C07C 205/12

(54) **Process for preparing 4-cyano-3-nitrobenzotrifluoride from 3-bromo-4-cyanobenzotrifluoride in the presence of catalytic cuprous cyanide and a phase transfer catalyst.**
Verfahren zur Herstellung von 4-Cyano-3-nitrobenzotrifluorid aus 3-Brom-4-cyanbenzotrifluorid in Anwesenheit einer katalytischen Menge an Kupfercyanid und eines Phasetransferkatalysators.
Procédé de préparation du 4-cyan-3-nitrobenzotrifluorure à partir du 3-bromo-4-cyanobenzotrifluorure en prèsence de cyanure cuivreux catalytique et un catalyseur de transfert de phase.

(43) Date of publication of application: 19.04.2000
(73) Proprietor: Bayer CropScience S.A., 69009 Lyon (FR)
(72) Inventor: Viauvy, Agnès, 69700 Saint Andeol Le Château (FR); Casado, Michel, 69360 St. Symphorien d'Ozon (FR)
(74) Representative: Rippel, Hans Christoph, Dr.

(56) References cited:
- EP-A- 0 110 559
- EP-A- 0 635 303
- EP-A- 0 758 643
- US-A- 2 195 076
- US-A- 3 890 326
- US-A- 4 886 936
- DATABASE WPI Section Ch, Week 8930 Derwent Publications Ltd., London, GB; Class B05, AN 89-216480 XP002096149 & JP 01 153669 A (IDEMITSU KOSAN CO LTD) , 15 June 1989
- J. MARCH: "Advanced Organic Chemistry" , JOHN WILEY & SONS , 1992 XP002104464 * page 659, 3-8 *
- BACON AND HILL: "Metal ions and complexes in organic reactions. Part I. Substitution Reactions between aryl halides and cuprous salts in organic solvents" JOURNAL OF THE CHEMICAL SOCIETY,1964, pages 1097-1107, XP002104463 London

## Description

This invention relates to novel processes for preparing intermediates (particularly 4-cyano-3-nitrobenzotrifluoride) useful in the preparation of pesticides.

Pesticidal 4-benzoylisoxazoles, particularly 5-cyclopropylisoxazole herbicides and intermediate compounds in their synthesis, are described in the literature, for example in European Patent Publication Nos. 0418175, 0487353, 0527036, 0560482, 0609798 and 0682659 and US patent 4886936.

Various methods for preparing these compounds are known. The present invention seeks to provide improved methods for the preparation of pesticides and the intermediate compounds useful in preparing them.

It is therefore an object of the present invention to provide novel and more economical processes for the preparation of ortho-nitrobenzonitrile compounds.

It is a further object of the present invention to provide processes for the preparation of ortho-nitrobenzonitrile compounds which proceed in high yield and/or with high selectivity.

It is a further object of the present invention to provide a process for the preparation of ortho-nitrobenzonitrile compounds which requires a low amount of copper compound as catalyst.

It is a further object of the present invention to provide a process for the preparation of ortho-nitrobenzonitrile compounds which proceeds using cuprous cyanide without the need for a catalyst.

It is a further object of the present invention to provide a process for the preparation of ortho-nitrobenzonitrile compounds which proceeds at a lower temperature than known methods and is therefore easier to perform.

The present invention allows these objects to be met in whole or in part.

### Summary of the Invention

The present invention accordingly provides a process for the preparation of an ortho-nitrobenzonitrile compound of formula (I): 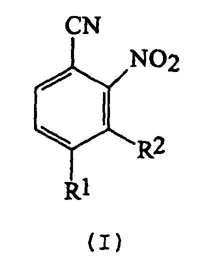 wherein:
R¹ represents trifluoromethyl;
and R² represents hydrogen; which process comprises the reaction of the corresponding ortho-nitrobromobenzene of formula (II): 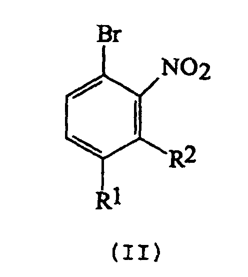
wherein R¹ and R² are as hereinbefore defined, with:
(b) an alkali metal cyanide in the presence of a catalytic amount of cuprous cyanide and a phase transfer catalyst.

Certain compounds of formula (I) are known and a number of processes for their preparation and conversion into herbicidal 4-benzoylisoxazole derivatives have been described in the European Patent Applications cited above.

Compounds of formula (II) are known or may be prepared by known methods.

In formulae (I) and (II) and in the formulae depicted hereinafter, preferred values of the symbols are as follows:-

R¹ represents trifluoromethyl and R² represents hydrogen.

### Detailed Description of the Invention

The above preparation (b) of compounds of formula (I) from compounds of formula (II), is performed using an alkali metal cyanide in the presence of a catalytic amount of cuprous cyanide and a phase transfer catalyst. Potassium cyanide is the preferred alkali metal cyanide. The amount of cuprous cyanide used is generally from 0.05 to 0.2 molar equivalents. The amount of alkali metal cyanide used is generally from 0.5-2 molar equivalents (preferably 0.7-1 molar equivalents). The phase transfer catalyst may be selected from tetraalkylammonium bromides or trialkylbenzylammonium bromides, in which the alkyl groups are straight- or branched-chain containing from one to six carbon atoms (such as tetramethylammonium bromide or benzyltrimethylammonium bromide); phosphonium salts (such as tributylhexadecylphosphonium bromide); guanidinium salts such as (hexabutylguanidinium bromide or hexamethylguanidinium bromide); and crown ethers (such as 18-crown-6). The amount of phase transfer catalyst used is generally from 0.05-0.3 molar equivalents.

Suitable solvents for the reaction include nitriles such as acetonitrile or benzonitrile; alcohols such as n-butanol; amides such as N,N-dimethylformamide or N-methylpyrrolidone; ketones such as methyl isobutyl ketone; esters such as methyl benzoate; ethers such as tetrahydrofuran or diglyme (diethylene glycol dimethyl ether); dimethylsulphoxide or sulpholane.

The concentration of the compound of formula (II) used in the reaction solvent is generally in the range from 0.1ml/mmol to 2ml/mmol, and preferably from 0.2ml/mmol to 1ml/mmol.

The reaction temperature is generally from 100°C to 200°C, preferably from 110°C to 160°C

According to a further feature of the present invention there is provided a process (c) for the preparation of a compound of formula (II) wherein R¹ and R² are as hereinbefore defined, which comprises the reaction of the corresponding compound of formula (II) wherein the bromine atom is replaced by a chlorine atom, with a bromide source.

Examples of suitable bromide sources include alkali metal bromides (such as potassium bromide or lithium bromide); alkaline earth metal bromides (such as magnesium bromide); cuprous bromide; cupric bromide; zinc bromide; hydrogen bromide; or bromine; or a mixture of lithium bromide and cuprous bromide. The preferred bromide source is a mixture of lithium bromide and cuprous bromide; or magnesium bromide or cupric bromide. The amount of bromide source used is generally one to five molar equivalents. When a mixture of lithium bromide and cuprous bromide is used, 0.1-1 molar equivalents of cuprous bromide is generally employed, together with one to two molar equivalents of lithium bromide.
A solvent is generally required to obtain good results. Suitable solvents for the reaction include nitriles such as acetonitrile or benzonitrile; ethers such as tetrahydrofuran or diglyme (diethylene glycol dimethyl ether); ketones such as methyl isobutyl ketone; esters such as methyl benzoate or n-butyl acetate; N-methylpyrrolidone; alkanoic acids such as acetic acid; dimethylsulphoxide and sulpholane.

The reaction temperature is generally from 100°C to 200°C, preferably from 130°C to 180°C. Good results are obtained when the process is carried out in a concentrated medium.
According to a further feature of the present invention process (b) can be combined with process (c) to prepare a compound of formula (I) starting from a compound of formula (II) wherein the bromine atom is replaced by a chlorine atom.

The compounds of formula (I) obtained by the processes of the present invention may be used in the preparation of herbicidally active 4-benzoylisoxazole derivatives for example according to the following reaction scheme:-

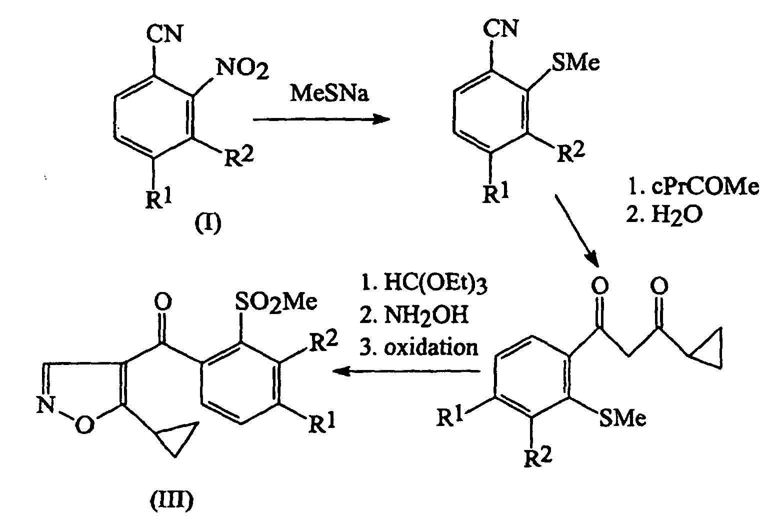

The 4-benzoylisoxazoles of formula (III) are described in for example European Patent Publication Nos. 0418175, 0527036 and 0560482.

The following non-limiting examples illustrate the invention. Where concentrations of ingredients in solvent are given these are understood to refer to the concentration of the compound of formula (II) in the solvent (i.e. ml of solvent/mmol of compound of formula (II)).

### Example 1

### Preparation of 4-cyano-3-nitrobenzotrifluoride from 4-bromo-3-nitrobenzotrifluoride using alkali metal cyanide and a catalytic amount of cuprous cyanide in the presence of a phase transfer catalyst (process (b)):

A mixture of 4-bromo-3-nitrobenzotrifluoride (1 equivalent), potassium cyanide (0.9 equivalent), cuprous cyanide (0.1 equivalent), and phase transfer catalyst (tetraethylammonium bromide or tetrabutylammonium bromide) was heated with benzonitrile, acetonitrile, n-butanol or dimethylsulphoxide at 110°C for 6 hours. The results (Table 1) show that an excellent selectivity may be obtained using benzonitrile, acetonitrile or n-butanol at 0.2ml/mmol or 0.4ml/mmol concentration. When a small amount of water (6 microlitres/mmol) was present in addition to the acetonitrile (at 0.4ml/mmol) the yield of title product was increased to 44% with selectivity remaining at a high level.

**Table 1**

| **Solvent** | **ml/mmol** | **Catalyst** | **eq** | **Conversion (%)** | **Yield (%)** | **Selectivity (%)** |
|---|---|---|---|---|---|---|
| PhCN | 0.2 | Et4NBr | 0.2 | 11 | 11 | ≥95 |
| PhCN | 0.2 | Bu4NBr | 0.2 | 11 | 11 | ≥95 |
| CH3CN | 0.2 | Et4NBr | 0.2 | 12 | 12 | ≥95 |
| CH3CN | 0.2 | Bu4NBr | 0.2 | 13 | 12 | ≥95 |
| CH3CN | 0.4 | Et4NBr | 0.1 | 17 | 18 | ≥95 |
| n-BuOH | 0.4 | Et4NBr | 0.1 | 15 | 15 | ≥95 |
| DMSO | 0.4 | Et4NBr | 0.1 | 92 | 32 | 35 |
| CH3CN | 0.4 (a) | Et4NBr | 0.1 | 49% | 44 | 89 |

| | | | | | | |
|---|---|---|---|---|---|---|
| (a) 6 microlitres of water/mmol were added to the acetonitrile in this experiment. | | | | | | |

### Example 2

### Preparation of 4-bromo-3-nitrobenzotrifluoride from 4-chloro-3-nitrobenzotrifluoride using various bromide sources:

A mixture of 4-chloro-3-nitrobenzotrifluoride (1 equivalent) and a bromide source (cuprous bromide, cupric bromide, lithium bromide or magnesium bromide) (1 equivalent), or a mixture of cuprous bromide (1 equivalent) and lithium bromide (1 equivalent) was heated for 6 hours at 160°C with benzonitrile, diglyme (diethylene glycol dimethyl ether), acetic acid or N-methylpyrrolidone (1ml/mmol) to give the title product. Table 2 shows that good selectivity may be obtained using various conditions and that the mixture of cuprous bromide and lithium bromide gives particularly good results. By comparison poor results were obtained in the absence of solvent.

**Table 2**

| **Solvent** | **Bromide** | **Conversion (%)** | **Yield (%)** | **Selectivity (%)** |
|---|---|---|---|---|
| PhCN | CuBr | 17 | 11 | 63 |
| diglyme | CuBr | 8 | 7 | 86 |
| PhCN | CuBr2 | 18 | 15 | 84 |
| diglyme | CuBr2 | 15 | 12 | 80 |
| AcOH | CuBr2 | 17 | 16 | 95 |
| diglyme | LiBr | 23 | 21 | 89 |
| NMP | LiBr | 17 | 9 | 51 |
| NMP | MgBr2 | 30 | 21 | 71 |
| NMP (a) | MgBr2 | 35 | 32 | 91 |
| PhCN | CuBr+LiBr | 51 | 49 | 95 |
| diglyme | CuBr+LiBr | 24 | 20 | 83 |
| AcOH | CuBr+LiBr | 36 | 31 | 86 |
| NMP | CuBr+LiBr | 32 | 17 | 52 |
| (b) | CuBr+LiBr | 2 | 1 | - |

| | | | | |
|---|---|---|---|---|
| (a) 0.1ml/mmol of NMP (N-methylpyrrolidone) were used in this experiment. | | | | |
| (b) no solvent used. | | | | |

### Example 3

### Preparation of 4-bromo-3-nitrobenzotrifluoride from 4-chloro-3-nitrobenzotrifluoride using a mixture of cuprous bromide and lithium bromide:

The above procedure of Example 2 was repeated using a mixture of cuprous bromide (1 equivalent) and lithium bromide (1 equivalent) with various concentrations of benzonitrile (0.02, 0.04, 0.1, 0.5, 1 or 2ml/mmol). Table 3 shows that good selectivity was obtained using all of these conditions and that when 0.04ml/mmol of benzonitrile was present the conversion, yield and selectivity were particularly good.

**Table 3**

| **PhCN ml/mmol** | **Conversion (%)** | **Yield (%)** | **Selectivity (%)** |
|---|---|---|---|
| 2 | 43 | 40 | 93 |
| 1 | 51 | 49 | 95 |
| 0.5 | 61 | 58 | 95 |
| 0.1 | 59 | 56 | 95 |
| 0.04 | 70 | 68 | 97 |
| 0.02 | 20 | 18 | 87 |

The above reaction was repeated but using diglyme (diethylene glycol dimethyl ether) instead of benzonitrile at various concentrations (0.01, 0.02, 0.04, 0.08, 0.1, 0.5, 1 or 2ml/mmol). Table 4 shows that the conversion, yield and selectivity were optimal at the 0.02-0.04ml/mmol concentration.

**Table 4**

| **Diglyme ml/mmol** | **Conversion (%)** | **Yield (%)** | **Selectivity (%)** |
|---|---|---|---|
| 2 | 24 | 18 | 75 |
| 1 | 24 | 20 | 83 |
| 0.5 | 25 | 20 | 81 |
| 0.1 | 40 | 39 | 97 |
| 0.08 | 31 | 29 | 95 |
| 0.04 | 52 | 50 | 96 |
| 0.02 | 50 | 48 | 96 |
| 0.01 | 34 | 33 | 97 |

The above reaction was repeated but using acetic acid, acetonitrile or tetrahydrofuran instead of benzonitrile at various concentrations (0.02, 0.04, 0.1 and 1ml/mmol). Table 5 shows the results, from which it may be seen that the optimal concentration depends upon the solvent, and that the best results were obtained at high concentration.

**Table 5**

| **Solvent** | **ml/mmol** | **Conversion (%)** | **Yield (%)** | **Selectivity (%)** |
|---|---|---|---|---|
| AcOH | 1 | 36 | 31 | 86 |
| AcOH | 0.1 | 39 | 36 | 91 |
| AcOH | 0.02 | 3 | 2 | - |
| CH3CN | 0.1 | 46 | 44 | 95 |
| CH3CN | 0.04 | 36 | 33 | 92 |
| CH3CN | 0.02 | 16 | 14 | 89 |
| THF | 0.1 | 47 | 40 | 84 |
| THF | 0.04 | 55 | 42 | 77 |
| THF | 0.02 | 43 | 38 | 89 |

### Example 4

### Preparation of 4-bromo-3-nitrobenzotrifluoride from 4-chloro-3-nitrobenzotrifluoride using various ratios of a mixture of cuprous bromide and lithium bromide:

A mixture of 4-chloro-3-nitrobenzotrifluoride (1 equivalent), cuprous bromide (1 equivalent) and lithium bromide (2 equivalents) in benzonitrile (4 equivalents) was heated at 180°C for 5 hours. The cooled mixture was extracted with toluene, washed with an aqueous solution of sodium bromide and with sodium bisulphite solution, and evaporated to give the title product. The conversion (based on 4-chloro-3-nitrobenzotrifluoride) was 84%, the yield 78%, and the selectivity 93%.

The above reaction was repeated using cuprous bromide (0.1 equivalent) and lithium bromide (2 equivalents) giving a conversion of 83% and a selectivity of 90%.

## Claims

1. A process for the preparation of a compound of formula (I): 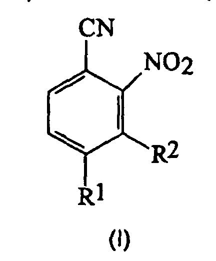 wherein:
R¹ represents trifluoromethyl and R² represents hydrogen which process comprises the reaction of the corresponding ortho-nitrobromobenzene of formula (II): 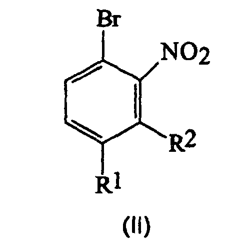 wherein R¹ and R² are as hereinbefore defined, with:
an alkali metal cyanide in the presence of a catalytic amount of cuprous cyanide and a phase transfer catalyst.

2. A process according to claim 1 in which 0.05-0.2 molar equivalents of cuprous cyanide is used.

3. A process according to claim 1 or 2 in which potassium cyanide is used.

4. A process according to claim 1, 2 or 3 in which the phase transfer catalyst is selected from tetraalkylammonium bromides, trialkylbenzylammonium bromides, phosphonium salts, guanidinium salts, and crown ethers.

5. A process according to any one of the preceding claims in which the concentration of the compound of formula (II) used in the reaction solvent is from 0.1ml/mmol to 2ml/mmol.

## Patentansprüche

1. Verfahren zur Herstellung einer Verbindung mit der Formel (I) : 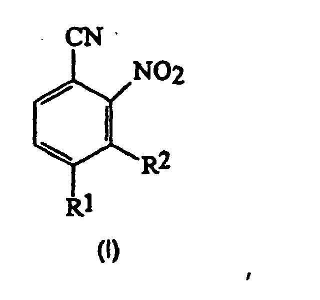 worin R¹ für Trifluormethyl steht und R² Wasserstoff darstellt, wobei das Verfahren die Umsetzung des entsprechenden ortho-Nitrobrombenzols mit der Formel (II): 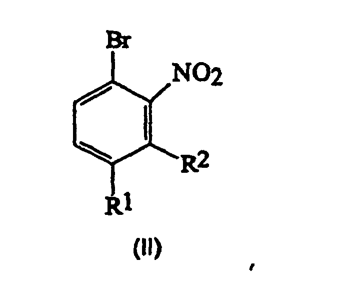 worin R¹ und R² wie vorstehend definiert sind, mit einem Alkalimetallcyanid in Gegenwart einer katalytischen Menge von Kupfer(I)cyanid und eines Phasentransferkatalysators umfaßt.

2. Verfahren nach Anspruch 1, worin 0,05 bis 0,2 Moläquivalente Kupfer(I)cyanid verwendet werden.

3. Verfahren nach Anspruch 1 oder 2, worin Kaliumcyanid eingesetzt wird.

4. Verfahren nach Anspruch 1, 2 oder 3, worin der Phasentransferkatalysator unter Tetraalkylammoniumbromiden, Trialkylbenzylammoniumbromiden, Phosphoniumsalzen, Guanidiniumsalzen und Kronenethern ausgewählt wird.

5. Verfahren nach einem der vorstehenden Ansprüche, worin die Konzentration der in dem Reaktionslösungsmittel verwendeten Verbindung der Formel (II) von 0,1 ml/mMol bis 2 ml/mMol beträgt.

## Revendications

1. Procédé pour la préparation d'un composé de formule (I) : 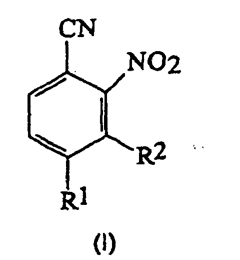 dans laquelle :
R¹ représente un groupe trifluorométhyle et R² représente un atome d'hydrogène, lequel procédé comprend la réaction de l'ortho-nitrobromobenzène correspondant de formule (II) : 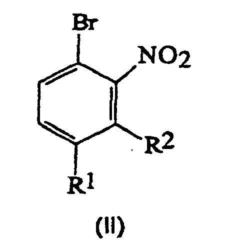 dans laquelle R¹ et R² sont tels que définis ci-dessus, avec :
un cyanure de métal alcalin en présence d'une quantité catalytique de cyanure cuivreux et d'un catalyseur de transfert de phase.

2. Procédé selon la revendication 1, dans lequel 0,05 à 0,2 équivalent molaire de cyanure cuivreux est utilisé.

3. Procédé selon la revendication 1 ou 2, dans lequel le cyanure de potassium est utilisé.

4. Procédé selon la revendication 1, 2 ou 3, dans lequel le catalyseur de transfert de phase est sélectionné parmi les bromures de tétraalkylammonium, les bromures de trialkylbenzylammonium, les sels de phosphonium, les sels de guanidinium et les éthers couronne.

5. Procédé selon l'une quelconque des revendications précédentes, dans lequel la concentration du composé de formule (II) utilisée dans le solvant de réaction est de 0,1 ml/mmol à 2 ml/mmol.
